(19) Europäisches Patentamt
European Patent Office
Office européen des brevets

(11) EP 1 149 820 B1

(12) **EUROPÄISCHE PATENTSCHRIFT**

(45) Veröffentlichungstag und Bekanntmachung des Hinweises auf die Patenterteilung:
**30.07.2003 Patentblatt 2003/31**

(51) Int Cl.[7]: **C07C 49/203**, C11B 9/00, A23L 1/226, A61K 7/46

(21) Anmeldenummer: **01109285.5**

(22) Anmeldetag: **17.04.2001**

(54) **2-,5-,6-,7-,8-Substituierte Oct-2-en-4-one**

2-,5-,6-,7-,8-substituted oct-2-ene-4-one

Oct-2-ène-4-one substitué en position 2,5,6,7,8

(84) Benannte Vertragsstaaten:
**AT BE CH CY DE DK ES FI FR GB GR IE IT LI LU MC NL PT SE TR**

(30) Priorität: **28.04.2000 CH 8502000**

(43) Veröffentlichungstag der Anmeldung:
**31.10.2001 Patentblatt 2001/44**

(73) Patentinhaber: **Givaudan SA**
**1214 Vernier (CH)**

(72) Erfinder: **Kraft, Philip**
**8600 Dübendorf (CH)**

(74) Vertreter: **Patentanwälte**
**Schaad, Balass, Menzl & Partner AG**
**Dufourstrasse 101**
**Postfach**
**8034 Zürich (CH)**

(56) Entgegenhaltungen:
**EP-A- 0 141 569** **US-A- 4 380 674**
**US-A- 4 448 713**

- **CHEMICAL ABSTRACTS, vol. 100, no. 13, 26. März 1984 (1984-03-26) Columbus, Ohio, US; abstract no. 102702, KIBINA, I. YU. ET AL: "Reactions of acetylenic 1,2,5-triols in an acidic medium" XP002172632 & DEPOSITED DOC. (1982), VINITI 4408-82, 12 PP. AVAIL.: VINITI,**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 149 820 B1

## Beschreibung

Die Erfindung betrifft 2-,5-,6-,7-,8-substituierte Oct-2-en-4-one, die Verwendung dieser Verbindungen als Riechstoffe oder Geschmacksstoffe sowie die damit hergestellten Riechstoff- oder Aromakompositionen.

Bis zur Entdeckung der Damascone genannten 1-(2,2,6-Trimethylcyclohexyl)-but-2-en-1-one war eine parfümistische Rekonstitution des Rosenöles quasi unmöglich [D. Kastner, *Parfuem. Kosmet.* **1985,** *66*, 5-16; D. Kastner, *Parfuem. Kosmet.* **1994**, *75*, 170-181]. Mit der Entdeckung der Damascone [G. Ohloff, E. Demole, J. *Chromatogr.* **1987**, *406*, 181-183] änderte sich aber nicht nur dies, sondern die Substanzklasse verlieh auch vielen anderen blumigen oder fruchtigen Akkorden Volumen, Frische und Natürlichkeit. Überdosen prägten darüber hinaus ganz neue Trends [M. Gras, *Perfum. Flavor.* **1990,** *15*, 25-28; M. Gras, *Perfum. Flavor.* **1992**, *17*, 1-12]. Daher hat es auch nicht an Versuchen gefehlt, Substanzen mit ähnlichen Geruchseigenschaften zu finden, von denen einige auch tatsächlich in die Parfümerie eingeführt wurden [G. Fräter, J. A. Bajgrowicz, P. Kraft, *Tetrahedron* **1998,** *54,* 7633-7703]. Diese Verbindungen sind jedoch strukturell den Damasconen sehr ähnlich, ja bis auf wenige Ausnahmen sogar konstitutionsisomer zu diesen. Ferner beschreibt die japanische Offenlegungschrift JP 55027135 3(4),4(5)-*diseco*- bzw. 2(3),4(5)-*diseco*-Damascone. Parfümistisch bieten alle diese Verbindungen wenig Neues, das heisst sie erweiterten das geruchliche Spektrum um die Stammverbindungen nur unwesentlich und kommen somit dem Bedarf an neuen innovativen Duftbausteine dieser Geruchsrichtung nicht nach. Durch die Strukturähnlichkeiten und das ähnliche Molekulargewicht haben die bekannten in die Parfümerie eingeführten Verbindungen mit damasconartigen Gerucheigenschaften sehr ähnliche Anwendungseigenschaften, also eine vergleichbare Diffusivität, Substantivität und Strahlungskraft in Kompositionen. Dem Bedarf nach innovativen neuen Duftbausteinen mit anderen Anwendungsbereichen kommen sie nicht wirklich nach. Diesen Mangel zu beheben und das geruchliche Spektrum der Damascone um neue Facetten zu erweitern, um damit neue Kompositionsmöglichkeiten für den Parfümeur und Aromatiker zu eröffnen, ist Aufgabe der Erfindung.

Es wurde nun überraschenderweise eine Gruppe von verzweigten acyclischen Alkenonen der allgemeinen Formel I,

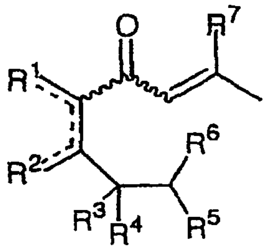

I

worin $R^1$, $R^2$ = $CH_2$, $CH_3$, $CHCH_3$, $CH_2CH_3$,

$R^3$ = H, $CH_3$, $CH_2CH_3$,

$R^4$, $R^5$, $R^6$, $R^7$ = H, $CH_3$,

bedeutet, mit der Bedingung, dass die Reste $R^4$, $R^5$, $R^6$, $R^7$ nicht alle zugleich Wasserstoff sind, und wobei eine gestrichelte Linie eine optionale Doppelbindung darstellt, gefunden, die die typischen Geruchseigenschaften der Damascone um neue, charakteristische Aspekte bereichern und völlig andere Anwendungseigenschaften besitzen, vor allem hinsichtlich einer viel größeren Diffusivität und Strahlungskraft. Einige dieser Verbindungen sind sogar erstaunlicherweise viel geruchsintensiver als die Damascone selbst.

Gemäss der allgemeinen Formel **I** muss somit nicht zwingend an den Positionen $R^1$(C-5), C-5(C-6) und $R^2$ (C-6) eine Doppelbindung vorhanden sein. (*E*/*Z*)-Isomere aller in einem Molekül vorhandenen Doppelbindungen (maximal zwei), sowie alle möglichen Stereoisomere werden von der allgemeinen Formel **I** umfasst, was durch die Darstellung mit Wellenlinien in den Formeln gezeigt wird.

Die erfindungsgemässen Verbindungen sind damit durch ein Oct-2-en-4-on-Skelett gekennzeichnet. Aufgrund der spezifischen Gruchs- und/oder Aromaeigenschaften sind Verbindungen mit einem 7-Methyloct-2-en-4-on-Skelett bevorzugt und Verbindungen mit einem (5*Z*)-7-Methylocta-2,5-dien-4-on-Skelett besonders bevorzugt.

Die allgemeine Formel **I** umfaßt somit die Verbindungen **1-9:**

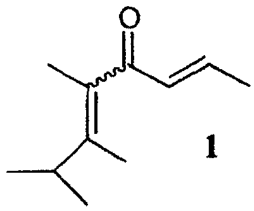
1

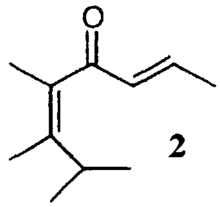
2

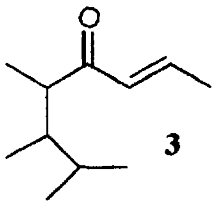
3

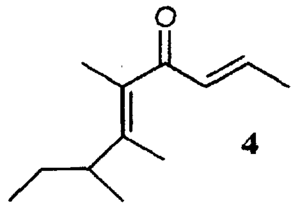
4

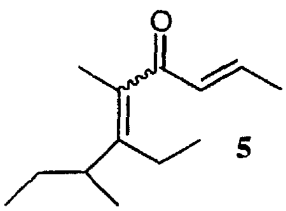
5

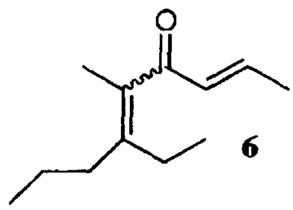
6

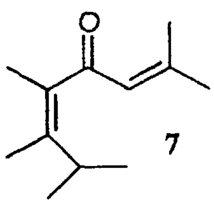
7

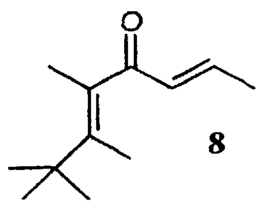
8

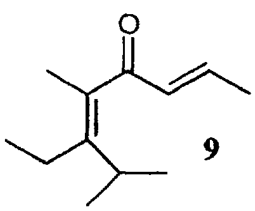
9

Die Verbindungen der allgemeinen Formel **I** eignen sich besonders zum Aufbau rosiger Akkorde, sowie allgemeinblumiger Buketts. Besonders hervorzuheben ist auch die Verwendung in fruchtigen Noten und moschusbetonten Kompositionen. Der Einsatz ist aber weder auf diese Akkorde beschränkt, noch auf spezielle Riechstoffe, Substanzklassen oder Geruchsnoten. Als Beispiele für weitere gut harmonierende Stoffklassen seien daher aufgeführt:

- Ätherische Öle und Extrakte, z. B. Ambrette Resinoid, Bergamottöl, Geraniumöl, Grapefruitöl, Mandarinenöl,

Patchouli-Öl, Rosen-Absolue, Sandelholzöl, Ylang-Ylang-Öl, Zitronenöl.

- Alkane, Alkene, Halogenverb. z. B. Famesen, *alpha*-Trichlormethylbenzylacetat.

- Alkohole, Ether, Acetale, z. B. Citronellol, Dihydromyrcenol, Ebanol®, Eugenol, Florol®, Geraniol, Helional®, *cis*-Hex-3-enol, Mayol®, Nerol, 2-Phenylethylalkohol, Rosenoxid, Sandalor®, Spirambren®.

- Aldehyde und Ketone, z. B. Adoxal®, Bourgeonal®, Cepionat, Cyclohexal®, Damascenon, *beta*-Dihydrojonon, Florhydral®, Hedion®, Himbeerketon (N-112), Hydroxycitronellal, Iso E Super®, Lemarom®, Lilial®, Methyljonon, 2-Methylundecanal, Myralden®, 10-Undecen-1-al, Undecanal, Vanillin, Vertofix®.

- Ester, Lactone, Nitrile, z. B. Allylamylglykolat, Benzylsalicylat, Citronellylacetat, Citronellylformiat, Cyclogalbanat®, Decalacton *gamma*, Gardenol®, Geranylacetat, *cis*-Hex-3-enylacetat, Hexylacetat, Linalylacetat, Phenylethylacetat, Peonil®, *gamma*-Undecalacton, Verdox®.

- Makro-, Poly-, Heterocyclen, z. B. Ambroxan®, Cashmeran®, Galaxolid®, Habanolid®, Thibetolid®.

Die folgenden Beispiele erläutern die Erfindung:

Beispiel 1

(2*E*,5*E*/*Z*)-5,6,7-Trimethylocta-2,5-dien-4-on (**1**)

(2*E*/*Z*)-2,3,4-Trimethylpent-2-ensäureethylester wurde gemäss der Vorschrift von H. Vieregge, H. M. Schmidt, J. Renema, H. J. T. Bos und J. F. Arends [*Recl. Trav. Chim. Pays-Bas* **1966,** *85*(9-10), 929-951] in 58 proz. Ausbeute durch Bortrifluorid-katalysierte Addition von Methyl-*iso*-propylketon an 1-Ethoxypropin hergestellt. Durch portionsweise Zugabe einer Lösung von 6.3 ml (73.9 mmol) 1-Bromprop-1-en in 60 ml trockenem Tetrahydrofuran zu 1.80 g (73.9 mmol) Magnesiumspänen unter stossweisem Erhitzen mit einer Heissluftpistole wurde unter Inertgasatmosphäre eine Grignard-Lösung bereitet. In einem weiteren Reaktionsgefäss stellte man unter Stickstoff bei -70 °C durch Zutropfen von 4.3 ml (42.7 mmol) 10 M Butyllithium-Lösung zu einer Lösung von 6.1 ml (42.7 mmol) Diisopropylamin in 22 ml trockenem Tetrahydrofuran und anschliessendem 10 min. Rühren bei dieser Temperatur eine Lithiumdiisopropylamid-Lösung her. Man liess dann bei Raumtemp. während 20 Minuten die zuvor hergestellte Grignard-Lösung und im Anschluss daran bei 35 °C wiederum während 20 Minuten eine Lösung von (2*E*/*Z*)-2,3,4-Trimethylpent-2-ensäureethylester in 90 ml trockenem Tetrahydrofuran gelöst zutropfen. Nach 2 stdg. Rühren bei 35 °C und 1 stdg. Erhitzen unter Rückfluss goss man den Ansatz auf Wasser und extrahierte das Produkt mit Ether. Die vereinigten etherischen Extrakte wurden mit Wasser und ges. NatriumchloridLösung gewaschen, über Natriumsulfat getrocknet und am Rotationsverdampfer eingeengt. Nach Flashchromatogrphie (Pentan / Ether, 19:1, $R_f$ = 0.22) erhielt man 1.8 g (25%) (2E,5*E*/*Z*)-5,6,7-Trimethylocta-2,5-dien-4-on (**1**) als charakteristisch riechende, farblose Flüssigkeit.

Geruch: Linear, sehr intensiv und sehr diffusiv, fruchtig-rosig, nach getrockneten Äpfeln, Pflaumen, Rosinen und anderen Trockenfrüchten; in der Kopfnote etwas rum- und caramelartig. - IR (Film): $\nu$ = 1653 $cm^{-1}$ ($\nu$ C=O, unges.), 1620 ($\nu$ C=C), 973 ($\delta$ C=C-H oop.), 1377 ($\delta$ $CH_3$).- $^1$H-NMR ($CDCl_3$): $\delta$ = 0.94 (d, *J* = 6.6 Hz, 6H, 7-$Me_2$, 5*E*), 1.01 (d, *J* = 6.8 Hz, 6H, 7-$Me_2$, 5*Z*), 1.54 (q, $^5J$ = 1.3 Hz, 3H, 6-Me, 2*E*, 5*Z*), 1.61 (q, $^5J$ = 0.97 Hz, 3H, 6-Me, 2*E*, 5*E*), 1.74 (q, $^5J$ = 0.97 Hz, 3H, 5-Me, 2*E*, 5*E*), 1.79 (q, $^5J$ = 1.3 Hz, 3H, 5-Me, *2E, 5Z*), 1.91-1.94 (m, 6H, 1-$H_3$, 5*E*+5*Z*), 2.57 (sept, *J* = 6.6 Hz, 1H, 7-H, 5*E*), 2.86 (sept, *J* = 6.8 Hz, 1H, 7-H, 5*Z*), 6.11 (dq, *J* = 15.9, 2.0 Hz, 1 H, 3-H, 2*E*, 5*Z*), 6.12 (dq, *J* = 15.6, 1.6 Hz, 1H, 3-H, 2*E*, 5*E*), 6.74 (dq, *J* = 15.9, 9.2 Hz, 1H, 2-H, 2*E*, 5*Z*), 6.77 (dq, *J* = 15.6, 6.8 Hz, 1H, 2-H, 2*E*, 5*E*). - $^{13}$C-NMR ($CDCl_3$): $\delta$ = 11.21 / 13.77 (2q, 5-Me), 15.02 / 15.91 (2q, 6-Me), 18.10 / 18.21 (2q, C-1), 19.97 / 20.39 (4q, 7-$Me_2$), 29.52 / 32.35 (2d, C-7), 128.69 / 128.81 (2s, C-5), 132.03 / 132.41 (2d, C-3), 140.32 / 140.47 (2s, C-6), 145.11 / 145.32 (2d, C-2), 201.57 / 201.77 (2s, C-4). - MS (EI): *m*/*z* (%) = 41 (43) [$C_3H_5$], 55 (30) [$C_4H_7$], 69 (32) [$C_4H_5O$], 123 (20) [$M^+$-$C_3H_7$], 136 (31) [$M^+$-2$CH_3$], 151 (100) [$M^+$-$CH_3$], 166 (11) [$M^+$].

Beispiel 2

(2*E*,5*Z*)-5,6,7-Trimethylocta-2,5-dien-4-on (**2**)

Durch Flashchromatographie (Pentan / Ether, 9:1, $R_f$ = 0.53) des (2*E*,5*E*/*Z*)-Gemisches **1** konnte eine Probe einheitlich (2*E*,5*Z*)-konfiguriertes 5,6,7-Trimethylocta-2,5-dien-4-on (**2**) als extrem intensiv riechende farblose Flüssigkeit isoliert werden, die die folgenden olfaktorischen und spektroskopischen Daten zeigte.

Geruch: Entspricht der Verbindung **1**, ist aber sehr viel intensiver und strahlender; Schwellenwert: 0.5 ng/l

Luft, das (5*E*)-Isomer zeigt hingegen einen Schwellenwert von 500 ng/l. - IR (Film): ν = 1653 $cm^{-1}$ (ν C=O, unges.), 1620 (ν C=C), 973 (δ C=C-H oop.), 1377 (δ $CH_3$). - $^1$H-NMR ($CDCl_3$): δ = 0.94 (d, *J* = 6.6 Hz, 6H, 7-$Me_2$), 1.61 (q, $^5J$ = 1.0 Hz, 3H, 6-Me, daher 5*Z*, das 5*E*-Isomer liegt bei 1.5 Hz), 1.74 (q, $^5J$ = 1.0 Hz, 3H, 5-Me, daher 5*Z*, das 5*E*-Isomer liegt bei 1.5 Hz), 1.92 (dd, *J* = 6.8, 1.6 Hz, 3H, 1-$H_3$), 2.57 (sept, *J* = 6.6 Hz, 1H, 7-H), 6.12 (dq, *J* = 15.6, 1.6 Hz, 1H, 3-H, daher 2*E*), 6.77 (dq, *J* = 15.6, 6.8 Hz, 1H, 2-H, daher 2*E*). - NOESY ($^1$H/$^1$H): 3-H/7-Me, 5-Me/6-Me. - 13C-NMR ($CDCl_3$): δ = 11.29 (q, 5-Me), 15.99 (q, 6-Me), 18.18 (q, C-1), 20.45 (2q, 7-$Me_2$), 32.41 (d, C-7), 128.85 (s, C-5), 132.47 (d, C-3), 140.45 (s, C-6), 145.41 (d, C-2), 201.94 (s, C-4). - MS (El): *m*/*z* (%) = 41 (41) [$C_3H_5$], 55 (30) [$C_4H_7$], 69 (31) [$C_4H_5O$], 123 (20) [$M^+$-$C_3H_7$], 136 (33) [$M^+$-2$CH_3$], 151 (100) [$M^+$-$CH_3$], 166 (11) [$M^+$].

Beispiel 3

(2*E*)-5,6,7-Trimethyloct-2-en-4-on (**3**)

20.0 g (117 mmol) eines Doppelbindungsisomeren-Gemisches des 2,3,4-Trimethylpent-2-ensäureethylesters [Darstellung: *siehe Beispiel 1*] wurden in 200 ml Essigester gelöst, mit 0.50 g (0.26 mmol, 0.2 mol-%) 10-prozentigem Platin auf Aktivkohle versetzt und bei Raumtemperatur 4 h in einem Autoklaven bei 25 bar Wasserstoff-Atmosphäre unter starkem Rühren hydriert. Der Katalysator wurde durch Abnutschen des Reaktionsgemisches über Celite abgetrennt. Nach Entfernen des Lösungsmittels am Rotationsverdampfer wurde im Vakuum über eine 10-cm Widmer-Kolonne destilliert, wobei beim Siedepunkt 105-108 °C / 100 mbar 16.9 g (83 %) farbloser 2,3,4-Trimethylvaleriansäureethylester erhalten wurden. Diese 16.9 g (98.1 mmol) wurden in 150 ml Ethanol / Wasser (1:1) gelöst und mit 9.60 g (147 mmol) 86-prozentigem Kaliumhydroxid versetzt. Das Reaktionsgemisch wurde 2 h zum Rückfluss erhitzt und nach dem Abkühlen mit 500 ml Wasser verdünnt und mit Salzsäure auf pH 1 gestellt. Die wässrige Phase wurde dreimal mit Ether extrahiert, die vereinigten organischen Phasen über Natriumsulfat getrocknet und am Rotationsverdampfer eingeengt. Es wurden 14.3 g rohe 2,3,4-Trimethylvaleriansäure erhalten, die in 250 ml trockenem Tetrahydrofuran gelöst wurden. Bei -10 °C wurden 9.8 ml (98 mmol) 10 M Butyllithium-Lösung in Hexan zugetropft. Nach 40 min. Rühren bei -10 °C ließ man bei dieser Temperatur 123 ml (98 mmol) 0.8 M Propenyllithium-Lösung in Ether langsam zutropfen. Nach 4 stündigem Rühren bei 30 °C wurde auf -10 °C abgekühlt und bei dieser Temperatur 16 ml (218 mmol) Aceton zutropfen gelassen. Nach weiteren 10 min. Rühren bei -10 °C wurden bei dieser Temperatur 200 ml gesättigte Ammoniumchlorid-Lösung zutropfen gelassen. Die Kühlung wurde entfernt und das Reaktionsgemisch mit 200 ml Wasser verdünnt. Die wässrige Phase wurde dreimal mit Ether extrahiert, die vereinigten organischen Extrakte über Magnesiumsulfat getrocknet und am Rotationsverdampfer eingeengt. Nach Flashchromatographie (Pentan / Ether, 19:1, $R_f$ = 0.24) an Kieselgel erhielt man 6.50 g (39%) (2*E*)-5,6,7-Trimethyloct-2-en-4-on als farblose, angenehm riechende Flüssigkeit.

Geruch: Schwächer als **1** und **2**, aber auch sehr diffusiv, fruchtig-rosig, nach Himbeeren und Trockenobst. - IR (Film): ν = 1629 $cm^{-1}$ (ν C=C), 1695 / 1668 (ν C=O, unges.), 970 (δ C=C-H oop.), 1378 (δ $CH_3$). - $^1$H-NMR ($CDCl_3$): δ = 0.70 / 0.74 / 0.76 / 0.84 (4d, *J* = 6.8 Hz, 6H, 7-$Me_2$), 0.92 / 0.93 (2d, *J* = 6.5 Hz, 3H, 6-Me), 0.98 / 1.05 (2d, *J* = 6.8 Hz, 3H, 5-Me), 1.49-1.71 (m, 1H, 7-H), 1.85-1.93 (m, 4H, 1-$H_3$, 6-H), 2.78 (quint, *J* = 6.8 Hz) / 2.61 (dq, *J* = 9.6, 6.8 Hz) [1H, 5-H], 6.21 (dq, *J* = 15.4, 1.6 Hz, 1H, 3-H), 6.85-6.94 (m, 1H, 2-H). - $^{13}$C-NMR ($CDCl_3$): δ = 11.03 / 11.69 (4q, 7-$Me_2$), 14.97 15.19 (2q, 5-Me), 18.08 / 18.22 (2q, C-1), 21.35 / 21.42 (2q, 6-Me), 27.10 / 30.30 (2d, C-7), 40.33 / 40.50 (2d, C-6), 46.73 / 47.70 (2d, C-5), 130.19 / 130.87 (2d, C-3), 141.83 / 142.19 (2d, C-2), 203.83 / 204.47 (2s, C-4). - MS (El): *m*/*z* (%) = 69 (100) [$C_4H_5O^+$], 83 (16) [$M^+$-$C_5H_{10}$-$CH_3$], 98 (58) [$M^+$-$C_5H_{10}$, *McLafferty*-Fragmentierung], 125 (2) [$M^+$-$C_3H_7$], 168 (1) [M+].

Beispiel 4

(2*E*,5*E*)-5,6,7-Trimethylnona-2,5-dien-4-on (**4**)

In Analogie zum Beispiel 1 und der allgemeinen Arbeitsvorschrift von H. Vieregge, H. M. Schmidt, J. Renema, H. J. T. Bos und J. F. Arends [*Recl. Trav. Chim. Pays-Bas* **1966,** *85*(9-10), 929-951] wurde 2,3,4-Trimethylhex-2-ensäureethylester durch Addition von 3-Methylpentan-2-on an 1-Ethoxypropin in 46 proz. Ausbeute hergestellt. 5.83 g (31.7 mmol) dieses 2,3,4-Trimethylhex-2-ensäureethylesters wurden in 100 ml Ethanol / Wasser (1:1) gelöst und darauf mit 2.93 g (44.4 mmol) 86-prozentigem Kaliumhydroxid versetzt. Nach 3 stündigem Erhitzen am Rückfluss wurden erneut 2.93 g (44.4 mmol) 86-prozentiges Kaliumhydroxid zugegeben und das Reaktionsgemisch darauf weitere 3h unter Rückfluss erhitzt. Nach dem Abkühlen fügte man 300 ml Wasser hinzu und säuerte mit konzentrierter Phosphorsäure an. Die wässrige Phase wurde mit 400 ml Ether extrahiert, die organischen Phasen getrocknet und am Rotationsverdampfer zur Trockne eingeengt Nach Flashchromatographie (Pentan / Ether, 4:1) an Kieselgel isolierte man 3.77 g (76%) 2,3,4-Trimethyl-hex-2-ensäure, die in 50 ml trockenem Tetrahydrofuran aufgenommen wurden. Bei -10 °C liess man unter Stickstoff 15.1 ml (24.2 mmol) 1.6 M Butyllithium-Lösung in Tetrahydrofuran zutropfen. Nach 15

min. Rühren bei dieser Temperatur liess man innerhalb von 10 min. 30.0 ml (24.2 mmol) Propenyllithium-Lösung in Ether zutropfen. Die Kühlung wurde entfernt und das Reaktionsgemisch auf 30 °C erwärmt. Bei dieser Tempratur liess man 1 h rühren, kühlte dann auf 0 °C ab und liess 4 ml Aceton gefolgt von 50 ml gesättigter Ammoniumchlorid-Lösung zutropfen. Nach dem Erwärmen auf Raumtemperatur verdünnte man das Reaktionsgemisch mit 200 ml Wasser und extrahierte das Produkt durch dreimaliges Waschen mit je 400 ml Ether. Die organischen Extrakte wurden vereinigt, getrocknet und am Rotationsverdampfer eingeengt, das Rohprodukt an Kieselgel mit 41 Pentan / Ether (49:1) und 21 Pentan / Ether (19:1) flashchromatographiert. Dadurch isolierte man 0.67 g (15%) (2*E*,5*E*)-5,6,7-Trimethylnona-2,5-dien-4-on (**4**) als farblose, intensiv und charakteristisch riechende Flüssigkeit.

Geruch: Linear, sehr intensiv und langanhaltend, fruchtig-rosig, nach Äpfeln und Trockenobst. - IR (Film): ν = 1652 $cm^{-1}$ (ν C=O, unges.), 972 (δ C=C-H oop.), 1620 (ν C=C), 1376 (δ $CH_3$). - $^1$H-NMR ($CDCl_3$): δ = 0.87 (t, *J* = 7.4 Hz, 3H, 9-$H_3$), 0.99 (d, *J* = 7.2 Hz, 3H, 7-Me), 1.38 (qd, *J* = 7.4, 7.2 Hz, 2H, 8-$H_2$), 1.50 (q, $^5J$ = 1.5 Hz, 3H, 6-Me), 1.79 (q, $^5J$ = 1.5 Hz, 3H, 5*E*-Me), 1.92 (dd, *J* = 6.8, 1.6 Hz, 3H, 1-$H_3$), 2.60 (sext, *J* = 7.2 Hz, 1H, 7-H), 6.11 (dq, *J* = 15.6, 1.6 Hz, 1H, 3-H), 6.80 (dq, *J* = 15.6, 6.8 Hz, 1H, 2*E*-H). - $^{13}$C-NMR ($CDCl_3$): δ = 12.17 (q, C-9), 13.63 (q, 5-Me), 15.35 (q, 6-Me), 18.16/18.23 (2q, C-1, 7-Me), 27.26 (t, C-8), 36.74 (d, C-7), 130.09 (s, C-5), 132.14 (d, C-3), 139.09 (s, C-6), 145.06 (d, C-2), 201.77 (s, C-4). - MS (EI): *m/z* (%) = 41 (73) [$C_3H_5$], 55 (33) [$C_4H_7$], 69 (62) [$C_4H_5O$], 109 (43) [$M^+$-$C_5H_{11}$], 123 (43) [$M^+$-$C_4H_9$], 136 (17) [$M^+$-$C_3H_8$], 151 (100) [$M^+$-$C_2H_5$], 165 (26) [$M^+$-CH3], 180 (13) [$M^+$].

Beispiel 5

(2*E*,5*E*/*Z*)-6-Ethyl-5,7-dimethylnona-2,5-dien-4-on (**5**)

Analog zum Beispiel 4 wurde aus Ethoxypropin und 4-Methylhexan-3-on 3-Ethyl-2,4-dimethylhex-2-ensäure hergestellt. 0.60 g (3.52 mmol) 3-Ethyl-2,4-dimethylhex-2-ensäure wurden in 10 ml trockenem Tetrahydrofuran bei -10 °C vorgelegt. Während 15 min injizierte man bei dieser Temperatur mit einer Spritze langsam 0.35 ml (3.5 mmol) 10M Butyllithium-Lösung, nach weiteren 45 min Rühren gab man 3.52 ml (3.52 mmol) 1M Propenyllithium-Lösung in Ether zu und liess 15h bei Raumtemperatur rühren. Danach kühlte man erneut auf - 10 °C ab und versetzte das Reaktionsgemisch bei dieser Temperatur mit 0.6 ml Aceton und nach 10 min Rühren mit 7 ml gesättigter Ammoniumchlorid-Lösung. Anschliessend wurde das Reaktionsgemisch auf 50 ml Wasser gegossen und das Produkt zweimal mit je 50 ml Ether extrahiert. Die Extrakte wurden vereinigt, mit je 50 ml Wasser und 25 ml gesättigter NatriumchloridLösung gewaschen, über Natriumsulfat getrocknet und am Rotationsverdampfer zur Trockne eingeengt. Nach Flashchromatographie (Pentan / Ether, 98:2, $R_f$ = 0.37) an Kieselgel isolierte man 200 mg (29%) farbloses, angenehm riechendes (2*E*,5*E*/*Z*)-6-Ethyl-5,7-dimethylnona-2,5-dien-4-on (**5**).

Geruch: Fruchtig-rosig, nach Trockenfrüchten, allerdings mit einer deutlichen karamell- und zuckergussartigen Nuance. - IR (Film): ν = 1652 $cm^{-1}$ (ν C=O, unges.), 973 (δ C=C-H oop.), 1623 (ν C=C), 1376 (δ $CH_3$).- $^1$H-NMR ($CDCl_3$): δ = 0.76 / 0.90 (t, *J* = 7.4 Hz, 3H, 9-$H_3$), 0.95 / 0.96 (2t, *J* = 7.2 Hz, 3H, $CH_3$, 6-Et), 1.06/1.08 (2d, *J* = 7.2 Hz, 3H, 7-Me), 1.26-1.50 (m, 2H, 8-$H_2$), 1.78/1.79 (2 br. s, 3H, 5-Me), 1.91 / 1.93 (2t, *J* = 1.6 Hz, 3H, 1-$H_3$), 1.98 / 2.05 (2 br. q, *J* = 7.2 Hz, 2H, $CH_2$, 6-Et), 2.24/2.55 (sext, *J* = 7.1 Hz, 1H, 7-H), 6.10 / 6.12 (2dq, *J* = 15.6, 1.6 Hz, 1 H, 3-H), 6.78 (br. dq, *J* = 15.6, 6.8 Hz, 1 H, 2*E*-H). - $^{13}$C-NMR ($CDCl_3$): δ = 12.22 / 12.48 (2q, C-9), 13.95 / 15.25 (2q, 5-Me), 15.73 / 15.79 (2q, $CH_3$, 6-Et), 18.12 / 18.18 / 18.64 / 18.77 (4q, C-1, 7-Me), 19.50 / 22.35 (2t, $CH_2$, 6-Et), 27.72 / 27.96 (2t, C-8), 37.49 / 40.24 (2d, C-7), 130.69 (2s, C-5), 132.08 / 132.64 (2d, C-3), 144.51 / 144.80 (2s, C-6), 144.91 / 145.17 (2d, C-2), 201.66 / 202.11 (2s, C-4). - MS (EI): *m/z* (%) = 41 (65) [$C_3H_5$], 55 (28) [$C_4H_7$], 69 (49) [$C_4H_5O$], 123 (35) [$M^+$-$C_5H_{11}$], 137 (76) [$M^+$-$C_4H_9$], 165 (100) [$M^+$-$C_2H_5$], 179 (25) [$M^+$-$CH_3$], 194 (5) [$M^+$].

Alle weiteren Verbindungen wurden in Analogie zu diesen Vorschriften hergestellt. Von ihnen sind daher nur die olfaktorische Charakterisierung und die spektroskopischen Daten aufgeführt.

Beispiel 6

(2*E*,5*E*/*Z*)-6-Ethyl-5-methylnona-2,5-dien-4-on (**6**)

Geruch: Fruchtig-rosig, nach Trockenfrüchten, mit einem leicht grünen Einschlag. - IR (Film): ν = 1652 $cm^{-1}$ (ν C=O, unges.), 972 (δ C=C-H oop.), 1376 (δ CH3). - $^1$H-NMR ($CDCl_3$): δ = 0.84 / 0.96 (2t, *J* = 7.4 Hz, 3H, 9-$H_3$), 0.95 / 1.03 (2t, *J* = 7.6, 3H, $CH_3$, 6-Et), 1.26-1.49 (m, 2H, 8-$H_2$). 1.78 / 1.79 (2 br. s, 3H, 5-Me), 1.91 / 1.93 (2dd, *J* = 1.6, 0.8 Hz. 3H, 1-$H_3$), 1.91-2.12 (m, 4H, C-7, $CH_2$, 6-Et), 6.12 / 6.14 (2dq, *J* = 15.8, 1.6 Hz, 1H, 3-H), 6.76 / 6.77 (2dq, *J* = 15.8, 7.0 Hz, 1H, 2*E*-H). - $^{13}$C-NMR ($CDCl_3$): δ = 12.33 / 13.03 (2q, C-9), 14.08 / 14.19 (2q, $CH_3$, 6-Et), 15.52 / 15.77 (2q, 5-Me), 18.19 (2q, C-1), 21.13 / 21.59 (2t, C-8), 23.91 / 26.34 (2t, $CH_2$, 6-Et), 32.44 / 35.23 (2t, C-7), 129.95 / 130.10 (2s, C-5), 132.17 / 132.21 (2d, C-3), 141.66 / 142.03 (2s, C-6), 145.04 / 145.15 (2d, C-2), 201.26 / 201.44 (2s, C-4). - MS (EI): *m/z* (%) = 41 (80) [$C_3H_5$], 55 (38) [$C_4H_7$], 69 (64) [$C_4H_5O$], 123 (100) [$M^+$-$C_4H_9$], 137 (31) [$M^+$-$C_3H_7$], 151 (93) [$M^+$-$C_2H_5$], 165 (41) [$M^+$-$CH_3$], 180 (11) [$M^+$].

Beispiel 7

(5*Z*)-2,5,6,7-Tetramethylocta-2,5-dien-4-on (**7**)

Geruch: Fruchtig-rosig, nach Trockenfrüchten, schwächer als Verbindung **2**, aber intensiver als das entsprechende (5*E*)-Isomer [(5*E*)-**7**]. - IR (Film): ν = 1612 $cm^{-1}$ (ν C=C), 1667 (ν C=O, unges.), 1378 (δ $CH_3$). - $^1$H-NMR ($CDCl_3$): δ = 0.96 (d, *J* = 6.8 Hz, 6H, 7-$Me_2$), 1.58 (q, $^5J$ = 0.8 Hz,3H, 6-Me), 1.75 (q, $^5J$ = 0.8 Hz, 3H, 5-Me), 1.91 (d, *J*= 1.4 Hz, 3H, 2*E*-Me), 2.15 (d, *J* = 1.1 Hz, 3H, 2*Z*-Me). 2.82 (sept, *J* = 6.8 Hz, 1H, 7-H), 6.09 (s, 1H, 3-H). - NOESY ($^1$H/$^1$H): 3-H/8-$H_3$, 1-H/3-H, 3-H/5-Me, 5-Me/6-Me, 6-Me/8-$H_3$. - $^{13}$C-NMR ($CDCl_3$): δ = 11.78 (q, 5-Me), 15.39 (q, 6-Me), 20.47 (2q, C-1, 2-Me), 27.58 / 27.63 (2q, 7-$Me_2$), 31.78 (d, C-7), 125.00 (d, C-3), 132.08 (s, C-5), 141.14 (s, C-6), 154.37 (s, C-2), 199.88 (s, C-4). - MS (EI): *m*/*z* (%) = 43 (26) [$C_3H_7$], 55 (52) [$C_4H_7$], 83 (59) [$C_5H_7O$], 123 (32) [$M^+$-$C_4H_9$], 137 (43) [$M^+$-$C_3H_7$], 150 (27) [$M^+$-2$CH_3$], 165 (100) [$M^+$-$CH_3$], 180 (20) [$M^+$].

Beispiel 8

(2*E*,5*E*)-5,6,7,7-Tetramethylocta-2,5-dien-4-on (**8**)

Geruch: Fruchtig, intensiv nach Apfel, aber auch stark nach Trockenfrüchten, mit einer etwas selerie- und jasmonartigen Seite, sowie agrestischen Aspekten. - IR (Film): ν = 1653 $cm^{-1}$ (ν C=O, unges.), 972 (δ C=C-H oop.), 1621 (ν C=C), 1377 (δ CH3). - $^1$H-NMR ($CDCl_3$): δ = 1.21 (s, 9H, 7-$Me_3$), 1.57 (q, *J* = 1.5 Hz, 3H, 6-Me, 5*E*), 1.88 (q, *J*= 1.5 Hz, 3H, 5*E*-Me), 1.93 (dd, *J*= 7.0, 1.6 Hz, 3H, 1-$H_3$), 6.04 (dq, *J* = 15.7, 1.6 Hz, 1H, 3-H, 2*E*), 6.74 (dq, *J* = 15.7, 6.8 Hz, 1H, 2*E*-H). - NOESY ($^1$H/$^1$H): 5-Me/7-$Me_3$, 6-Me/7-$Me_3$.- $^{13}$C-NMR ($CDCl_3$): δ = 17.80 (q, 5-Me), 18.29 (q, C-1), 19.11 (q, 6-Me), 29.82 (3q, 7-$Me_3$), 35.88 (s, C-7), 130.43 (s, C-5), 131.92 (d, C-3), 140.35 (s, C-6), 145.73 (d, C-2), 203.15 (s, C-4). - MS (EI): *m*/*z* (%) = 41 (81) [$C_3H_5$], 57 (76) [$C_4H_9$], 69 (65) [$C_5H_9$], 109 (38) [$M^+$-$C_5H_{11}$], 124 (30) [$M^+$-$C_4H_8$], 137 (24) [$M^+$-$C_3H_7$], 150 (9) [$M^+$-2$CH_3$], 165 (100) [$M^+$-$CH_3$], 180 (10) [$M^+$].

Beispiel 9

(2*E*,5*Z*)-6-Ethyl-5,7-dimethylocta-2,5-dien-4-on (**9**)

Geruch: Fruchtig-rosig, nach Himbeeren und Trockenfrüchten, intensiver, diffusiver und agrestischer als das entsprechende (5*E*)-Isomer [(5*E*)-**9**], das allerdings mehr nach frischen Äpfeln riecht. - IR (Film): ν = 1651 $cm^{-1}$ (ν C=O, unges.), 972 (δ C=C-H oop.), 1621 (ν C=C), 1376 (δ $CH_3$). - $^1$H-NMR ($CDCl_3$): δ = 0.94 (d, *J* = 6.8 Hz, 6H, 7-$Me_2$), 1.06 (t, *J* = 7.6 Hz, 3H, $CH_3$, 6-Et), 1.77 (s, 3H, 5-Me), 1.93 (dd, *J*= 6.8, 1.6 Hz, 3H, 1-$H_3$), 2.07 (q, *J* = 7.6 Hz, 2H, $CH_2$, 6-Et), 2.54 (sept, *J* = 6.8 Hz, 1H, 7-H), 6.11 (dq, *J* = 15.7, 1.6 Hz, 1H, 3-H, daher 2*E*), 6.77 (dq, *J* = 15.7, 6.8 Hz, 1H, 2-H, daher 2*E*). - NOESY ($^1$H/$^1$H): 5-Me/$CH_2CH_3$. - $^{13}$C-NMR ($CDCl_3$): δ = 14.08 (q, 5-Me), 15.64 (q, $CH_3$, 6-Et), 18.15 (q, C-1), 19.32 (t, $CH_2$, 6-Et), 20.82 (2q, 7-$Me_2$), 32.94 (d, C-7), 129.41 (s, C-5), 132.55 (d, C-3), 145.37 (d, C-2), 145.55 (s, C-6), 202.26 (s, C-4). - MS (EI): *m*/*z* (%) = 41 (80) [$C_3H_5$], 55 (29) [$C_4H_7$], 69 (61) [$C_5H_9$], 81 (17) / 95 (17) / 109 (24) / 123 (40) / 137 (76) / 151 (36) / 165 (100) [$M^+$-$C_nH_{2n+1}$], 180 (14) [$M^+$].

Beispiel 10

Blumig-fruchtig-grüner Damen-Duft mit Verbindung **1**

| Nr. | Verbindung / Bestandteil | Gewichtsanteile in ‰ |
|---|---|---|
| 1. | Adoxal | 3 |
| 2. | Ambrofix, 10-proz. in BB | 15 |
| 3. | Benzylacetat, extra | 6 |
| 4. | Bergamottöl | 80 |
| 5. | Calone 1951 | 4 |
| 6. | Cedrylmethylether | 5 |
| 7. | Citronellylacetat | 15 |
| 8. | Citronellol, extra | 35 |

(fortgesetzt)

| Nr. | Verbindung / Bestandteil | Gewichtsanteile in ‰ |
|---|---|---|
| 9. | Dimetol | 2 |
| 10. | DPG (Dipropylenglykol) | 94 |
| 11. | Estragol | 1 |
| 12. | Ethyllinalool | 35 |
| 13. | Ethyl-3-methyl-3-phenylglycidat (Erdbeer-Aldehyd) | 1 |
| 14. | Eugenol, reinst | 3 |
| 15. | Floralozon [3-(4-Ethylphenyl)-2,2-dimethylpropanal], 10-proz. in DPG | 4 |
| 16. | Floropal (2,4,6-Trimethyl-4-phenyl-1,3-dioxan) | 3 |
| 17. | Galaxolid 50 PHT (4,6,6,7,8,8,-Hexamethyl-1,3,4,6,7,8-hexahydrocyclopenta[g] benzopyran) | 140 |
| 18. | Gardenol (Essigsäure-1-phenylethylester) | 30 |
| 19. | Hedion (Methyldihydrojasmonat) | 120 |
| 20. | cis-3-Hexenylacetat, 10-proz. in DPG | 17 |
| 21. | cis-3-Hexenylsalicylat | 9 |
| 22. | Iso E Super | 60 |
| 23. | Isoraldein 70 (Methyljonon) | 35 |
| 24. | Lavendelöl, französich | 7 |
| 25. | Lilial [2-Methyl-3-(4-tert-butylphenyl)propanal] | 90 |
| 26. | Melonal (2,6-Dimethyl-5-hepten-1-al), 10-proz. in DPG | 12 |
| 27. | 3-(4-Methoxyphenyl)-2-methylpropanal | 6 |
| 28. | 1-Phenyl-2-methylprop-2-ylacetat | 9 |
| 29. | Sandalor (5-(2,2,3-Trimethyl-3-cyclopenten-1-yl)-3-methylpentan-2-ol] | 15 |
| 30. | Tagettesöl, extra | 3 |
| 31. | Terpineol | 10 |
| 32. | Tropional [2-Methyl-3-(3,4-methylendioxyphenyl)propanal] | 48 |
| 33. | Viridin (2-Phenylacetaldehyddimethylacetal) | 1 |
| 34. | Ylang-Ylangöl | 2 |
| 35. | Zitronenöl, italienisch | 30 |
| 36. | Verbindung **1**, 10-proz. in DPG | 50 |
| | | 1000 |

Diese Komposition ergibt ein feminines, blumig-aquatisches Parfüm mit hesperidisch-grünem Kopf aus Bergamott-, Zitrus- und Lavendel-Noten, rosig, maiglöckchen- und wassermelonen-betontem Herz und fruchtig-moschusartigholziger Basisnote.

Die Verbindung **1** vereinigt sich harmonisch mit dem Rosen-Akkord und hebt ihn wie auch die fruchtigen Aspekte dieser Kreation hervor. Sie integriert darüberhinaus harmonisch auch die lavendelartigen Facetten im Angeruch. Im Vergleich mit Damasconen und ihren Analoga werden die hesperidischen Elemente viel stärker herausgehoben, und die Verbindung **1** verleiht darüberhinaus dem Duft eine sehr viel grössere Diffusivität als dies beim Einsatz von Damasconen der Fall wäre.

Beispiel 11

Parfümkomposition mit Verbindung **1** für eine Cremeseife

| Nr. | Verbindung / Bestandteil | Gewichtsanteile in 1/1120 |
|---|---|---|
| 1. | Benzylacetat, extra | 10 |

(fortgesetzt)

| Nr. | Verbindung / Bestandteil | Gewichtsanteile in 1/1120 |
|---|---|---|
| 2. | Benzylsalicylat | 4 |
| 3. | Citronellol, extra | 200 |
| 4. | Dimethylsulfid, 1% in Triethylcitrat | 0.1 |
| 5. | Ethylvanillin | 0.4 |
| 6. | Eugenol, pur | 2.5 |
| 7. | Geraniol, extra | 110 |
| 8. | Geranium, Bourbon RGV | 20 |
| 9. | Geranylacetat, pur | 10 |
| 10. | cis-3-Hexenol | 1 |
| 11. | Irisantheme [3-Methyl-4-(2,6,6-trimethyl-2-cyclohexen-1-yl)-3-buten-2-on] | 60 |
| 12. | Isopulegol | 2 |
| 13. | Kamillenöl, römisch | 1 |
| 14. | Koavone | 3 |
| 15. | Laurylaldehyd | 1 |
| 16. | Nerol | 90 |
| 17. | Nonanal | 1.5 |
| 18. | 2-Noninsäuremethylester | 0.5 |
| 19. | 2-Phenylacetaldehyd, 85-proz. in 2-Phenylethylalkohol | 2 |
| 20. | 2-Phenylessigsäure, pur, kristallin | 0.5 |
| 21. | 2-Phenylethylalkohol | 420 |
| 22. | Phenethylphenylacetat | 0.5 |
| 23. | 1-Phenyl-2-methyl-2-propanylacetat | 6 |
| 24. | Rosacetol (alpha-Trichloromethylbenzylacetat) | 6 |
| 25. | Rose-de-Mai, RHS MEF | 2 |
| 26. | Rosenoxid | 1 |
| 27. | Zimtalkohol, synthetisch | 15 |
| 28. | Verbindung **1** , 1-proz. in DPG | 150 |
| | | 1120 |

Diese Komposition ergibt ein fruchtig-rosiges Parfümöl, angelehnt an den typischen Duft der bulgarischen Rose.

Die Verbindung **1** stellt einen zentralen Dufteckpfeiler in dieser betont rosigen Komposition dar. Sie verleiht der Kreation einen sehr viel natürlicheren Charakter als die Damascone, die den Gesamteindruck im direkten Vergleich mehr in Richtung Pflaume, Holz- und Weinnote verschieben. Die Verbindung **1** bringt darüberhinaus mehr apfelartige, grüne Aspekte in die Komposition ein und arbeitet so den mild-reinigenden, pflegenden Produktcharakter der Cremeseife besser heraus.

## Patentansprüche

1. Verbindungen der allgemeinen Formel **I**

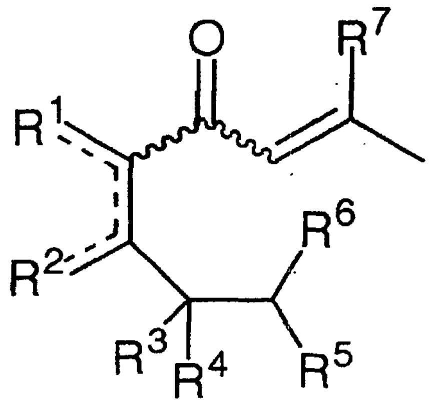

I

, worin

$R^1$, $R^2$ = $CH_2$, $CH_3$, $CHCH_3$, $CH_2CH_3$,

$R^3$ = H, $CH_3$, $CH_2CH_3$,

$R^4$, $R^5$, $R^6$, $R^7$ = H, $CH_3$, bedeutet, mit der Bedingung, dass die Reste $R^4$, $R^5$, $R^6$, $R^7$ nicht alle zugleich Wasserstoff sind, und wobei eine gestrichelte Linie eine optionale Doppelbindung darstellt, einschliesslich der (*E*/*Z*)-Isomere aller Doppelbindungen und aller möglichen Stereoisomere, ausgenommen $(CH_3)_2C = CHCOC(CH_3) = C(CH_3)CH_2CH_3$.

2. Verbindungen der allgemeinen Formel **I** gemäss Anspruch 1 mit einer (*E*)-konfigurierten Doppelbindung in Position 5(6).

3. Verbindungen der allgemeinen Formel **I** gemäss Anspruch 1, in denen $R^4$ = $CH_3$ ist.

4. Verbindungen der allgemeinen Formel **I** gemäss Anspruch 1 mit einer (*E*)-konfigurierten Doppelbindung in Position 5(6) und in denen $R^4$ = $CH_3$ ist.

5. Eine Verbindung der allgemeinen Formel **I** gemäss Anspruch 1 aus der Gruppe (2*E*,5*Z*)-5,6,7-Trimethylocta-2,5-dien-4-on, (2*E*)-5,6,7-Trimethyloct-2 -en-4-on, (2*E*,5*Z*)-5,6,7-Trimethylnona-2,5-dien-4-on, (2*E*,5*E*/*Z*)-6-Ethyl-5,7-dimethylnona-2,5-dien-4-on, (2*E*,5*E*/*Z*)-6-Ethyl-5-methylnona-2,5-dien-4-on, (2*E*,5*Z*)-2,5,6,7-Tetramethylocta-2,5-dien-4-on, (2*E*,5*E*)-5,6,7,7-Tetramethylocta-2,5-dien-4-on, (2*E*,5*Z*)-6-Ethyl-5,7-dimethylocta-2,5-dien-4-on und aller ihrer Doppelbindungsisomere sowie (2*E*,5*E*/*Z*)-5,6,7-Trimethylocta-2,5-dien-4-on.

6. Riechstoff-oder Aromakomposition, **gekennzeichnet durch** einen Gehalt mindestens einer Verbindung nach einem der Ansprüche 1 bis 5.

7. Verwendung mindestens einer Verbindung der allgemeinen Formel I

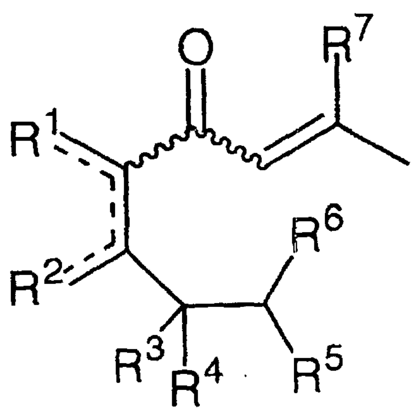

I

worin

$R^1$, $R^2$ = $CH_2$, $CH_3$, $CHCH_3$, $CH_2CH_3$,
$R^3$ = H, $CH_3$, $CH_2CH_3$,
$R^4$, $R^5$, $R^6$, $R^7$ = H, $CH_3$,

bedeutet, mit der Bedingung, dass die Reste $R^4$, $R^5$, $R^6$, $R^7$ nicht alle zugleich Wasserstoff sind, und wobei eine gestrichelte Linie eine optionale Doppelbindung darstellt, einschliesslich der (*E*/*Z*)-Isomere aller Doppelbindungen und aller möglichen Stereoisomere, als Riech- oder Geschmacksstoff.

## Claims

1. Compounds having the general formula I

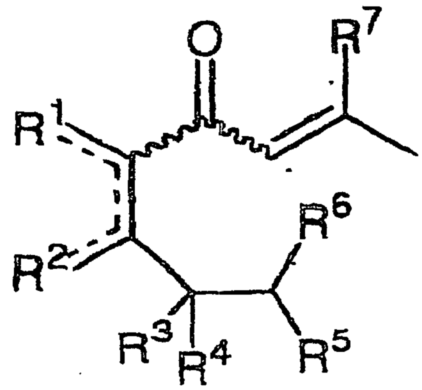

I

wherein

$R^1$, $R^2$ = $CH_2$, $CH_3$, $CHCH_3$, $CH_2CH_3$,
$R^3$ = H, $CH_3$, $CH_2CH_3$,
$R^4$, $R^5$, $R^6$, $R^7$ = H, $CH_3$,

subject to the condition that the radicals $R^4$, $R^5$, $R^6$, $R^7$ are not all simultaneously hydrogen, and wherein a chain line indicates an optional double compound, including the (*E*/*Z*) isomers of all double bonds and all possible stereoisomers, except for $(CH_3)_2C{=}CHCOC\ (CH_3)\ {=}C\ (CH_3)CH_2CH_3$.

2. Compounds having the general formula I according to claim 1, with an (*E*) configured double bond in position 5(6).

3. Compounds having the general formula I according to claim 1, in which $R^4$ = $CH_3$.

4. Compounds having the general formula I according to claim 1 with an (*E*)-configured double bond in position 5(6) and in which $R^4$ = $CH_3$.

5. A compound having the general formula I according to claim 1, out of the group (*2E,5Z*)-5,6,7-trimethylocta-2,5-dien-4-one, (*2E*)-5,6,7-trimethyloct-2-en-4-one, (*2E,5Z*)-5,6,7-trimethylnona-2, 5-dien-4-one, (*2E,5E*/*Z*)-6-ethyl-5,7-dimethylnona-2,5-dien-4-one, (*2E,5E*/*Z*)-6-ethyl-5-methylnona-2,5-dien-4-one, (*2E,5Z*)-2,5,6,7-tetramethylocta-2,5-dien-4-one, (*2E,5E*)-5,6,7,7-tetramethylocta-2,5-dien-4-one, (*2E,5Z*)-6-ethyl-5-7-dimethylocta-2,5-dien-4-one and all their double-bond isomers and *(2E,5E/Z)-5,6,7-trimethylocta-2,5*-dien-4-one.

6. A perfume or flavouring composition **characterised by** a content of at least one compound according to any of claims 1 to 5.

7. Use of at least one compound having the general formula I

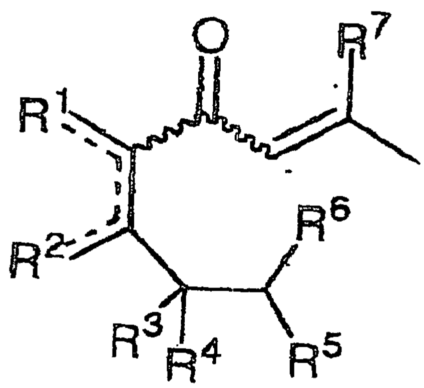

I

wherein $R^1$, $R^2$ = $CH_2$, $CH_3$, $CHCH_3$, $CH_2CH_3$,
$R^3$ = H, $CH_3$, $CH_2CH_3$,
$R^4$, $R^5$, $R^6$, $R^7$ = H, $CH_3$,
subject to the condition that the radicals $R^4$, $R^5$, $R^6$, $R^7$ are not all simultaneously hydrogen, and wherein a chain line indicates an optional double bond, including the (*E*/*Z*) isomers of all double bonds and all possible stereoisomers as a perfume or flavouring.

## Revendications

1. Composés de formule générale **I**

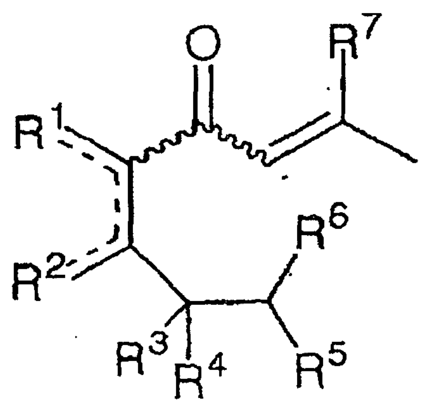

I

dans laquelle $R^1$, $R^2$ = $CH_2$, $CH_3$, $CHCH_3$, $CH_2CH_3$,
$R^3$ = H, $CH_3$, $CH_2CH_3$,
$R^4$, $R^5$, $R^6$, $R^7$ = H, $CH_3$
à la condition que les résidus $R^4$, $R^5$, $R^6$, $R^7$ ne soient pas tous simultanément de l'hydrogène, et où une ligne en tirets représente une double liaison optionnelle, y compris les isomères-(*E*/*Z*) de toutes les doubles liaisons et tous les stéréoisomères possibles, à l'exclusion de $(CH_3)_2C = CHCOC(CH_3) = C(CH_3)CH_2CH_3$.

2. Composés de formule générale **I** selon la revendication 1 ayant une double liaison configurée en (*E*) en position 5(6).

3. Composés de formule générale **I** selon la revendication 1 dans lesquelles $R^4$ = $CH_3$.

4. Composés de formule générale **I** selon la revendication 1 ayant une double liaison configurée en (*E*) en position 5(6) et dans lesquelles $R^4$ = $CH_3$.

5. Composés de formule générale **I** selon la revendication 1, choisi dans le groupe (*2E*, *5Z*)-5,6,7-triméthylocta-2,5-diène-4-one, (*2E*)-5,6,7-triméthyloct-2-ène-4-one, (*2E*, *5Z*) -5,6,7-triméthylnona-2,5-diène-4-one, (*2E,5E*/*Z*)-6-éthyl-5,7-diméthylnona-2,5-diène-4-one, (*2E,5E*/*Z*) -6-éthyl-5-méthylnona-2,5-diène-4-one, (*2E, 5Z*)-2,5,6,7-tétraméthylocta-2,5-diène-4-one, (*2E,5E*)-5,6,7,7-tétraméthylocta-2,5-diène-4-one, (*2E*, *5Z*)-6-éthyl-5,7-diméthylocta-2,5-diène-4-one et tous leurs isomères à double liaison ainsi que (*2E,5E*/*Z*)-5,6,7-triméthylocta-2, 5-diène-4-one.

**6.** Composition de substance odorante ou d'arôme, **caractérisée par** une teneur en au moins un composé selon l'une des revendications 1 à 5.

**7.** Utilisation d'au moins un composé de formule générale I

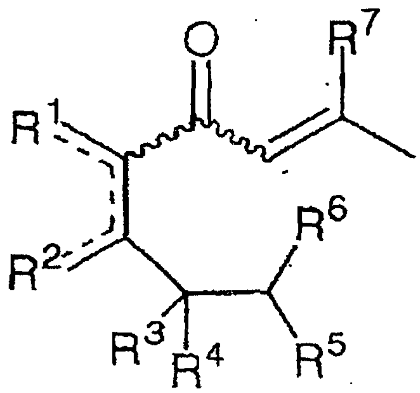

**I**

dans laquelle $R^1$, $R^2$ = $CH_2$, $CH_3$, $CHCH_3$, $CH_2CH_3$,
$R^3$ = H, $CH_3$, $CH_2CH_3$,
$R^4$, $R^5$, $R^6$, $R^7$ = H, $CH_3$
à la condition que les résidus $R^4$, $R^5$, $R^6$, $R^7$ ne soient pas tous simultanément de l'hydrogène, et où une ligne en tirets représente une double liaison optionnelle, y compris les isomères- (*E*/*Z*) de toutes les doubles liaisons et tous les stéréoisomères possibles, en tant que substance odorante ou de sapidité.